## Europäisches Patentamt

## European Patent Office

## Office européen des brevets

(19)

(11) Publication number: **0 028 902**
**B1**

(12) **EUROPEAN PATENT SPECIFICATION**

(45) Date of publication of patent specification: **18.05.83**

(21) Application number: **80303835.5**

(22) Date of filing: **29.10.80**

(51) Int. Cl.³: **C 12 N 5/00, C 12 N 15/00**
**//C12P1/00, A61K39/395**

(54) **Method for the preferential formation of hybrid cells making a desired antibody.**

(30) Priority: **01.11.79 US 90130**

(43) Date of publication of application:
**20.05.81 Bulletin 81/20**

(45) Publication of the grant of the patent:
**18.05.83 Bulletin 83/20**

(84) Designated Contracting States:
**CH DE FR GB IT LI**

(56) References cited:
NATURE, vol. 256, 7th August 1975, pages 495—497 G. KOHLER et al.: "Continuous cultures of fused cells secreting antibody of predefined specificity"
NATURE, vol. 212, 8th October 1966, pages 156—157 J. R. BATTISTO et al.: "Dual immunological unresponsiveness induced by cell membrane coupled hapten or antigen"
THE JOURNAL OF IMMUNOLOGY, vol. 122, no. 5, May 1979, pages 1899—1904 U. S. A. D. H. SHERR et al.: "Immune suppression in vivo with antigenmodified syngeneic cells. II. T Cell-mediated nonresponsiveness to Fowl-gamma-globulin"

(73) Proprietor: **BIO-RESPONSE INC.**
**550 Ridgefield Road**
**Wilton Connecticut (US)**

(72) Inventor: **Rose, Sam**
**2244 Webster Street**
**San Francisco California 94115 (US)**

(74) Representative: **Marsh, Roy David et al,**
**Brewer & Son 5-9, Quality Court Chancery Lane**
**London WC2A 1HT (GB)**

(56) References cited:
THE JOURNAL OF IMMUNOLOGY, vol. 116, no. 3, March 1976, pages 623—626 U. S. A. W. LIANG et al.: "Resistance to murine leukemia in mice rejecting syngeneic somatic hybrid cells"
CHEMICAL ABSTRACTS, vol. 88, no. 17, 24th April 1978, page 394, no. 119299m Columbus, Ohio, U.S.A. G. KOHLER et al.: "Fusion of T and B cells"
CHEMICAL ABSTRACTS, vol. 87, no. 9, 29th August 1977, page 262, no. 64990b Columbus, Ohio, U. S. A. G. POSTE et al.: "Fusion of mammaliam cells by lipid vesicles"

THE JOURNAL OF IMMUNOLOGY, vol. 122, no. 2, February 1979, pages 585—591 U. S. A. L. D. LESERMAN et al.: "Binding of antigen-bearing fluorescent liposomes to the murine myeloma tumor MOPC 315"

BIORESERACH INDEX, vol. 39, no. 3, abstract no. 3467 Federation Proceedings, 1980 U. S. A. A. E. HODACH et al.: "Mono clonal antibodies to tumor cell surface antigens lymphocyte hybridomas obtained by antigen directed cell fusion"

Method for the preferential formation of hybrid cells making a desired antibody.

The present invention relates to the field of biochemistry, and more particularly, to the production of monoclonal antibodies.

Antibodies, which are specific immunoglobulin molecules, harvested from an immunized subject, are mixed with other immunoglobulins. The ratio of specific antibody to other immunoglobulin molecules defines its purity and can vary from 1:10 to 1:10,000. It is difficult, and sometimes impossible, to isolate the required antibody from other immunoglobulins. For most uses, the value of specific antibody is increased as their purity increases. It is, in part, for these reasons that hybridization of antibody producing cells or their precursors (lymphocytes) with cancer cells has been attempted in order to obtain a large, homogeneous population of cells which are hybrids of both a cancer cell and an antibody producing cell, and have some of the characteristics of both these cells.

Functional hybrid cells are made by fusing or hybridizing cells of the same differentiation path, for example, malignant (cancer) myeloma B cells with normal lymphoid B cells, some of which synthesize specific antibody because the lymphoid B cells are obtained from antigen stimulated animals. G. Köhler et al., *Somatic Cell Genetics*, 3, p. 303 (1977); R. A. Goldsberg, et al., *Nature*, 267, p. 707 (1977); G. J. Hammerling, et al., *European J. Immunology*, 1, p. 743 (1977); R. Hyman and T. Kelleher, *Somatic Cell Genetics*, 1, p. 355 (1975); P. Coffino, et al., *Nature New Biol.*, 231, p. 87 (1971). Some of the resultant hybrid cells have the desirable features of both of the parents from which they arose. Like the normal antibody producing cell parent, some of the hybrids can synthesize the specific antibody. Like the malignant myeloma parent, the hybrids can divide continuously to produce a homogeneous tumor cell population called a hybridoma. The combination of these two agreeable characteristics gives the hybridomas the potential for producing unlimited amounts of monoclonal antibody.

The conventional hybridization process can be described as having four steps. Step (1) Cell-attaching step. In the first step, the parent cells are caused to randomly and non-specifically attach to one another to form doublets. This first step is achieved by adding polyethylene glycol or inactivated Sendai Virus to the mixture of normal and malignant cells. Polyethylene glycol or Sendai Virus cause cells to randomly and non-specifically stick or attach to one another thus forming a series of doublets. See M. L. Gefter, et al., *Somatic Cell Genetics*, 3, 231 (1977); G. Köhler and C. Milstein, *European J. Immunology*, 6, p. 511 (1976); R. Tees, *Research in Immunology*, Academic Press, N. Y. 1976. Step (2) Cell hybridization step. In the second step a proportion of these doublets spontaneously form hybrids by a mechanism which is not understood. Because of the random non-specific nature of the first cell attaching step, only a portion of the hybrids arising from the second step are specific antibody producers. (Actually it turns out that the proportion of specific hybrids is some 100 times higher than expected from the known proportion of specific versus non-specific normal lymphoid cells in the original lymphoid population obtained from the antigen stimulated animal. This is explained by postulating that the antigen stimulated lymphoid cells hybridize at a higher frequency than non-stimulated lymphoid cells.) Step (3) Isolating hybrid cells. In the third step, hybrid cells of the normal and malignant myeloma cells are isolated from normal cells, malignant myeloma cells, hybrids of normal and normal cells or hybrids of malignant and malignant cells by culturing the mixed population of cells in a medium in which only the hybrids of normal and malignant cells will grow. This isolation step is achievable by virtue of the fact that mutants of the myeloma cells having particular characteristics are used as the malignant parent of the hybrid. These characteristics are then present in the subsequently-formed hybrid and this, together with a culture medium of particular composition, allows only such hybrids having both the characteristics of the mutant malignant and the normal cell to survive and grow G. Köhler, *Nature*, 256, p. 495 (1975). Step (4) Isolating antibody producing hybrids. In the fourth step hybrids producing the required specific antibody must be isolated from all other hybrids. Notwithstanding the higher than expected frequency of antibody producing hybrids, it is still necessary to isolate the required malignant-normal hybrids making the specific antibody from malignant-normal hybrids not making this antibody. This isolation, depending on the special circumstances, can be tedious, difficult, or impossible. To achieve a relatively high frequency of specific hybrids, the normal cells must be collected from an organism which is stimulated with antigen by a regime which generally causes most of the cells to be making antibody of low binding affinity. For this reason, most of the antibody-making hybrids make antibodies of low binding affinity which are not as valuable as high binding antibodies for most diagnostic or therapeutic procedures. It is very difficult to isolate the few hybrids making high binding affinity antibody from the many hybrids making low affinity antibody. These isolation difficulties are serious limitations to the widespread use of hybridomas for the manufacture of antibodies for use in medicine.

A known method of hybridization includes collecting lymphocytes from mice which were previously immunized with the appropriate antigen. A large number of lymphocytes from the immunized subject are collected and they are then mixed with mutant cancer myeloma cells having a particular and hybrid isolation exploitable characteristic and incubated with polyethylene glycol or with a UV irradiated Sendai Virus, or the cells are centifuged in the presence of lecithin which tends to break down the cell membrane.

These hybridizations develop as a consequence of cells non-specifically attaching to each other. By this means, approximately 50 to 100 hybrid cells would be formed for about every 10,000 normal cells. However, from these 50 to 100 hybrids, perhaps only one to five cells would be the desired hybrid, *i.e.*, a hybrid of a cancer cell and a cell which produces the desired antibody. The number of negative hybrids (i.e., those hybrids not making the desired antibody) produced is very high, because there is no natural affinity between the lymphocytes and the myeloma cancer cells, these cells being made to attach to one another by polyethylene glycol or Sendai Virus. One of the known methods used to isolate the desired hybrid cells making the desired antibody is to clone all of the hybrid cells in multiples of ones, tens, hundreds, thousands, or millions, and then testing the fluid of each of these populations for their antibody content. Those populations of cells whose culture fluid contains antibody are further sub-cloned and this process is repeated until a single hybrid cell making the desired antibody is isolated and from this single cell, the homogeneous hybridoma mass is formed. However, this is an extremely time consuming and difficult process.

The present invention alleviates the problems mentioned above. Hybridization by the method of this invention produces a higher proportion of hybrids making the desired antibody compared to hybrids not making the desired antibody.

The object of the invention is to increase the frequency ratio of desired specific hybrids to undesired hybrids by arranging that the first cell attaching step occurs as a consequence of there being a specific affinity attachment between the two parents making up the hybrids, namely, the antibody-producing cell and the myeloma, rather than using agents such as polyethylene glycol or Sendai Virus which cause non-specific attachment of the cells.

According to the process of this invention, a method of hybridization is provided which depends upon arranging that there be a specific and uniquely selective attachment between the antibody-producing cells and the myeloma cells. Hybridization following such specific and selective attachment will lead to a high proportion of the formed hybrids being specific antibody producers. This specific attachment step is achieved by providing that there be antigen specific to the desired antibody on the surface of the malignant myeloma cells which form the malignant parent of the hybrid. The hybridization process can be described using the four-step format earlier described. In step 1 the myeloma cells with the antigen on their surface are mixed, without the addition of polyethylene glycol or Sendai Virus, with normal lymphoid cells obtained from an animal or subject responding to the same antigen. Some of these normal lymphoid cells will have specific receptors for the antigen which is present on the myeloma cells. Lymphoid cells with specific receptors for the antigen will become specifically attached to the antigen on the surface of the myeloma cells to form doublets. In step 2, a proportion of such doublets will spontaneously hybridize. In step 3, hybrids are isolated by their growth in hybrid selecting media. In step 4, hybrids making specific antibody are isolated. Because no polyethylene glycol or Scindia Virus is used in step 1, this method greatly increases the proportion of hybrids which are specific antibody producers, and this in turn makes their isolation much easier.

The above method has been tested for phthalate antigen and has been found to be highly successful. Ninety percent of all hybrids formed by the specific attachment method were specific antibody producers.

New and improved methods for the formation of hybrid cells are described in detail hereinafter. These hybrid cells are formed from myeloma tumor cells and antibody producing cells or their precursors and have agreeable characteristics of both types of cells. These hybrids cells will divide continuously and will produce antibodies which can be used to diagnose various conditions, including a malignant condition or to treat various conditions, including a cancer.

In accordance with the process of this invention, a population of normal cells comprised of cells producing the desired antibody or the precursors to these cells are obtained. The population of cells can be obtained, for example, by one of the following methods:

(a) immunizing an organism with the antigen specific to the desired antibody and isolating the cells from the organism (e.g., by collecting the lymphocytes from the immunized organism);

(b) isolating the cells produced by an organism in response to a disease state which exhibits the antigen specific to said desired antibody. For example, a tumor-bearing or tumor-cured organism will have antibody producing cells making antibody against the tumor antigen;

(c) stimulating normal cells of an organism *in vitro* with the antigen specific to the desired antibody; and

(d) treating normal cells of an organism *in vitro* with a chemical capable of stimulating the normal cells to produce the full complement of antibodies which the cells of that organism are genetically able to produce, including the desired antibody. Examples of such chemicals are lipopolysaccharides and various plant lectins.

Mutant myeloma cells which have the antigen specific to the desired antibody on their surface or as part of their surface are provided. These cells can be obtained, for example, by natural occurrence or by one of the following methods:

(a) attaching the antigen to the surface of the myeloma cell covalently through chemical attachment without killing or severely injuring the myeloma cell. Battisto, J. R. and Bloom, B. R., *Nature*, 212, p. 156 (1966);

(b) incubating myeloma cells with the antigen to bring about adsorption of the antigen on the surface of the myeloma cells. Sherr, D. H. et al., *J. Immunology*, 122, p. 1899 (1979). This can also be accomplished by culturing myeloma cells with an organism (such as trypanasomes) which releases the antigen into the culture medium for attachment to the myeloma cells;

(c) utilizing bridging means whereby the antigen is attached to a molecule, such as a fatty acid, e.g., palmytolic acid, which has a high natural affinity for cell membranes;

(d) incorporating the antigen onto or within a liposome (micro-spheres with, e.g., a lipid outer layer and a central aqueous core) which has a natural tendency to fuse with cell membranes and to deposit the products of the liposome onto the cell surface membrane;

(e) incubating myeloma cells which have receptors for complexes with complexes or fluid containing complexes of the antigen and antibody. The complexes attach to the myeloma cells through the Fc region of the antibody. If the antigen is multi-valent and the complex is in the region of antigen excess, the attached complex will have free antigen available for attachment to the specific antibody producing cell. Berken, A., *J. Expt. Med.*, 123, p. 119 (1966); and

(f) inducing the myeloma cells to have the antigen on their surface. This can be accomplished by infecting the myeloma cell with an organism (e.g., virus or parasite) whose antigen becomes part of the surface of the myeloma cells, or which induces the myeloma cell to make a specific antigen.

A particular method for inducing the myeloma cells to have the desired antigen on their surface is to introduce the gene apparatus of a particular cell having that antigen into the myeloma cell. The resultant myeloma will have the desired surface antigen. Thus, the required antigen on the myeloma cell can be induced by first hybridizing the myeloma cells to, say, the melanoma cell having the desired antigen. This first hybridization would be achieved in the standard way using polyethylene glycol or Sendai Virus, and from previous data it can be predicted that the hybrid would exhibit the receptor specific for malanoma. Liang, W. and Cohen, E. P., *J. Immunology*, 116, p. 623 (1976). This hybrid can then be mixed *without* polyethylene glycol or Sendai Virus to a population of lymphoid cells from a tumor bearing or tumor immunized subject. There will be specific cell attachment between the first hybrid, now having melanoma antigen on its surface, and those normal lymphoid cells having the specific receptor for the melanoma antigen.

The hybridization process is then carried out in accordance with earlier-described steps using normal lymphoid cells and myeloma cells having antigen on their surface in the absence of polyethylene glycol or Sendai Virus taking advantage of the specific coupling between the myeloma cells and those normal lymphoid cells having specific receptor sites for the antigen on the surface of the myeloma cells.

The presence of the melanoma gene apparatus could prevent the final cell which contains three gene systems (myeloma, melanoma and antibody producing lymphoid cell) from functioning as an antibody producer. See, e.g., Köhler, G., et al., *Somatic Cell Genetics*, 3, p. 303 (1977). Several modifications of the above triple method may obviate this potential difficulty. In the first modification, the strategy consists of first irradiating the melanoma cell at a level which would cause cell death, not immediately, but at the next cell division. The irradiated melanoma is hybridized to myeloma using polyethylene glycol. The hybrids are isolated by the hybrid selective medium. These hybrids are now mixed (without polyethylene glycol) with the immunized lymphoid cell population containing cells making anti-melanoma antibody.

Since the first hybrid will exhibit melanoma antigen, it should specifically couple and then hybridize with the specific anti-melanoma antibody producing cell. After coupling, the cells are again placed in hybrid selecting medium. The melanoma cell genetic component of the first hybrid will die (chromosome dysfunction) as a consequence of the irradiation, and thus only those first hybrids which have *also* hybridized to normal lymphoid cells will survive in hybrid selective medium. Many of these should be hybrids between myeloma cells and anti-melanoma antibody producing cells. These latter hybrids should survive and be functional in their ability to make anti-melanoma antibody.

In another modification of the above-described method, the first hybridization is made between cells of different species, e.g., melanoma cells from a man and myeloma cells from mouse. It is known that such hybrids are unstable in that there is a progressive loss of human chromosomes. These "unstable hybrid" cells are now (that is, while the human chromosomes are still present and therefore human antigens including melanoma antigens are expressed) hybridized to mouse antibody producing cells without polyethylene glycol. The resultant "triple" hybrids may not produce antibody because of the inhibitory effect of the melanoma gene component. However, since the latter is derived from human, it will progressively be lost and therefore its inhibitory effect on antibody production will also be eliminated. The resultant mouse myeloma-mouse antibody producing cell hybrid will be relatively stable and be able to produce the anti-melanoma antibody.

Continuing with the process of this invention, the population of cells comprised of antibody producing cells or the precursors of these cells are then mixed, e.g., incubated, with the myeloma cells which have the desired antigen on their surface. Specific coupling will occur between these two populations and hybridization will follow this coupling to produce a high frequency of hybrids producing the desired antibody. The hybrids and the specific hybrids are then isolated by the hybrid selecting medium and by sub-cloning and testing.

High affinity antibodies are particularly valuable for diagnosis and therapy. The usual immunizing regime, in order to collect the lymphocytes containing antibody producing cells, for high frequency hybridizing, usually results in most of the cells being producers of antibody of low affinity. Thus, though the frequency of specific hybridization is high (thereby facilitating the selection of specific hybrids), most of the resulting hybrids produce antibody of low binding affinity. The present invention provides two techniques for producing a relatively high frequency of hybrid cells making high affinity antibody.

One method is to immunize the organism with an antigen regimen which is known to induce the production of high affinity antibody producing cells. However, the presence of these cells cannot be exploited using the conventional hybridization method because the immunization regime is such that there would be a low frequency of specific hybridization, and, therefore, it would be very difficult to isolate the desired hybrid. Using the method of the present invention, this problem is obviated because though the absolute number of specific hybrids is low, as in the case above, so too is absolute number of non-specific hybrids produced. Thus, there is no difficulty in isolating the desired hybrids.

The second method exploits the fact that the first step in hybridization (i.e., cell-attachment) is immunologically specific and is, therefore, capable of being influenced in a specific way. The method allows the automatic and selective formation of hybrids making only high affinity antibody. The first cell-sticking step involves immunological specificity. From known immunological data it can be predicted that the addition of specific antibody or antigen to the mixture of the myeloma cells with antigen on their surface and normal antibody producing cells having a receptor for that antigen will competitively inhibit the specific first cell-sticking step and will therefore reduce the frequency of hybridization. If various conditions are chosen, such as cell concentrations, antigen and antibody concentration and binding affinity of antibody, virtually all coupling between these two cell types will be prevented and all hybridization also thus will be prevented. Conditions could be chosen so that coupling only occurs between the myeloma cells with antigen on their surface and the antibody producing cells having antigen receptors of the highest binding affinity. Thus, the process will automatically generate, virtually, only hybrids made from normal lymphoid parents which have high-binding antigen receptors and which therefore make high-binding antibody. These hybrids should, by reflecting the normal parent, also make high affinity antibody.

Example I

Myeloma cells with antigen attached are mixed, without polyethylene glycol or Sendai Virus, with normal lymphoid cells, some of which are antibody producing cells. The myeloma cells with antigen attached and the specific antibody producing cells will attach to each other to form doublets. A proportion of these doublets will form hybrids which are then isolated. Because no polyethylene glycol or virus is added, virtually all the hybrids formed will be hybrids making the desired specific antibody. This reduces to a negligible value the effort required in isolating the desired hybrids.

Example II

Myeloma cells with antigen attached are mixed with normal lymphoid cells, some of which are antibody producing cells, in the presence of added antibodies or antigens. The concentration and other parameters of all three substances are controlled so that all specific cell attachments will be inhibited except those between the antibody producing cells which have the highest binding affinity receptors. These hybrids are then subsequently isolated.

Example III

Balb C mice were immunized with phthalate antigen and these provided the lymphocytes for the subsequent hybridization step. Phthalate antigen was attached to dextran which itself is attached to palmytolic acid and this complex was then adsorbed onto myeloma cells. The attachment occurs because it is known that this fatty acid has a tendency to attach to cell surface membranes. The fatty acid thus acts as a bridge between the myeloma and the antigen. The normal antibody producing cells and the myeloma with antigen attached were mixed and incubated together. The entire cell population was then placed in a medium which will only support the growth of hybrids between the normal and the myeloma cells. After being placed in the hybrid selecting medium, the cells were distributed into 200 microculture chambers. The number of cells in each chamber is such that approximately one hybrid will form.

per chamber. One-hundred and eighty of these chambers contained an anti-phthalate antibody producing cell. These positive hybrids were then innoculated into Balb C mice and from the single hybrid injected into the mouse a homogeneous population of tumor cells developed. The blood from these hybridization-bearing mice contained a large quantity of monoclonal antibody.

## Claims

1. A method for the preferential formation of hybrid cells making a desired antibody, comprising the steps of: providing a population of normal cells comprised of cells producing the desired antibody or the precursors to these cells; providing myeloma cells; mixing together said population of normal cells and said myeloma cells for a time sufficient to bring about attachment and hybridization between said myeloma cells and said cells producing the desired antibody or the precursors to the cells; and isolating the hybrids which are produced in the previous step and which produce the desired specific antibody; characterised in that said myeloma cells have the antigen specific to said desired antibody on their surface or as part of their surface.

2. A method as claimed in claim 1, characterized in that said isolation step comprises isolating all hybrids between said myeloma cells and said normal cells and then isolating therefrom those hybrids producing said desired antibody.

3. A method as claimed in either claim 1 or claim 2, characterized in that said population of normal cells comprised of cells producing the desired antibody or the precursors to these cells is obtained by a method selected from one of:

(a) immunizing an organism with the antigen specific to the desired antibody and isolating the cells from said organism;

(b) isolating the cells produced by an organism in response to a diseased state which exhibits the antigen specific to said desired antibody;

(c) stimulating normal cells of an organism *in vitro* with the antigen specific to the desired antibody; and

(d) treating normal cells of an organism *in vitro* with a chemical capable of stimulating said normal cells to produce the desired antibody.

4. A method as claimed in any one of the preceding claims, characterized in that said myeloma cells are obtained by attachment of said antigen to the surface of a normal myeloma cell by a method selected from one of:

(a) attaching said antigen to the surface of said normal myeloma cells through covalent chemical attachment;

(b) contacting said normal myeloma cells with said antigen to bring about adsorption of said antigen on the surface thereof;

(c) attaching the antigen to a bridging means capable of attachment to cell surface membranes;

(d) incubating said normal myeloma cells with complexes of the antigen and the desired antibody to bring about an attachment between the myeloma and the complex; and

(e) incorporating the antigen onto or within a liposome which fuses with the membrane of said normal myeloma cell to deposit the products of the liposome on the myeloma cell surface.

5. A method as claimed in claim 1, characterized in that said myeloma cells are provided by inducing normal myeloma cells to have on their surface the required antigen.

6. A method as claimed in claim 5, characterized in that said inducing step comprises hybridizing the normal myeloma cell with a cancer cell having the antigen specific to the desired antibody.

7. A method as claimed in claim 1, characterized in that either antigen or specific antibody are also mixed with said population of normal cells and myeloma cells in said mixing step, thereby to inhibit all specific attachment between the myeloma cells and the cells producing the desired antibody except between myeloma cells and those cells which produce the desired antibody and which have the highest affinity for the antigen present on said myeloma cells.

## Patentansprüche

1. Verfahren zur bevorzugten Herstellung von Hybridzellen, die einen gewünschten Antikörper bilden, das die folgenden Stufen umfaßt: Erzeugung einer Population von normalen Zellen, bestehend aus Zellen, die den gewünschten Antikörper oder die Vorläufer für diese Zellen bilden; Erzeugung von Myelomzellen; Vermischen der Population der normalen Zellen mit den Myelomzellen für eine Zeitspanne, die ausreicht, um eine Bindung und Hybridisierung zwischen den Myelomzellen und den Zellen, die den gewünschten Antikörper oder die Vorläufer für diese Zellen bilden, herbeizuführen; und Isolierung der Hybride, die in der vorangegangenen Stufe entstanden sind und den gewünschten Antikörper bilden, dadurch gekennzeichnet, daß die Myelomzellen das für den gewünschten Antikörper spezifische Antigen auf ihrer Oberfläche oder als Teil ihrer Oberfläche besitzen.

2. Verfahren nach Anspruch 1, dadurch gekennzeichnet, daß die Isolierungsstufe umfaßt das Isolieren aller Hybride zwischen den Myelomzellen und den normalen Zellen und das anschließende Isolieren derjenigen Hybride daraus, die den gewünschten Antikörper bilden.

3. Verfahren nach Anspruch 1 oder 2, dadurch gekennzeichnet, daß die Population von normalen Zellen, bestehend aus Zellen, die den gewünschten Antikörper oder die Vorläufer für diese Zellen bilden, nach einen Verfahren erzeugt wird, das aus folgenden ausgewählt wird:

a) Immunisieren eines Organismus mit dem für den gewünschten Antikörper spezifischen Antigen und Isolieren der Zellen aus dem Organismus;

b) Isolieren der von einem Organismus als Folge eines Krankheitszustandes gebildeten Zellen, die das für den gewünschten Antikörper spezifische Antigen aufweisen;

c) Stimulieren der normalen Zellen eines Organismus in vitro mit dem für den gewünschten Antikörper spezifischen Antigen; und

d) Behandeln von normalen Zellen eines Organismus in vitro mit einer Chemikalie, die in der Lage ist, die normalen Zellen zur Bildung des gewünschten Antikörpers zu stimulieren.

4. Verfahren nach einem der vorhergehenden Ansprüche, dadurch gekennzeichnet, daß die Myelomzellen erhalten werden durch Binden des Antigens an die Oberfläche einer normalen Myelomzelle nach einem Verfahren, das aus folgenden ausgewählt wird:

a) Binden des Antigens an die Oberfläche der normalen Myelomzellen durch eine kovalente chemische Bindung;

b) Inkontaktbringen der normalen Myelomzellen mit dem Antigen, um eine Adsorption des Antigens an der Oberfläche derselben herbeizuführen;

c) Binden des Antigens an eine Brückenbildungseinheit, die zu einer Bindung an Zelloberflächenmembranen befähigt ist;

d) Inkubieren der normalen Myelomzellen mit Komplexen aus dem Antigen und dem gewünschten Antikörper, um eine Bindung zwischen dem Myelom und dem Komplex herbeizuführen; und

e) Inkorporieren des Antigens auf oder in ein Liposom, das mit der Membran der normalen Myelomzelle verschmilzt unter Abscheidung der Produkte des Liposoms auf der Myelomzellenoberfläche.

5. Verfahren nach Anspruch 1, dadurch gekennzeichnet, daß die Myelomzellen derart erzeugt werden, daß normale Myelomzellen so induziert werden, daß sie auf ihrer Oberfläche das erforderliche Antigen aufweisen.

6. Verfahren nach Anspruch 5, dadurch gekennzeichnet, daß die Induzierungsstufe umfaßt die Hybridisierung der normalen Myelomzelle mit einer Krebzelle, die das für den gewünschten Antikörper spezifische Antigen aufweist.

7. Verfahren nach Anspruch 1, dadurch gekennzeichnet, daß das Antigen oder der spezifische Antikörper auch mit der Population aus den normalen Zellen und den Myelomzellen in der Mischstufe gemischt wird, um dadurch jegliche spezifische Bindung zwischen den Myelomzellen und den Zellen, welche den gewünschten Antikörper bilden, mit Ausnahme derjenigen zwischen Myelomzellen und den Zellen, die den gewünschten Antikörper bilden und die höchste Affinität für das auf den Myelomzellen vorhandene Antigen aufweisen, zu verhindern.

## Revendications

1. Procédé pour la formation préférentielle de cellules hybrides fabriquant un anticorps désiré, comprenant les étapes consistant à fournir une population de cellules normales constituées de cellules produisant l'anticorps désiré ou des précurseurs de ces cellules; à fournir des cellules du myélome; à mélanger ensemble ladite population de cellules normales et lesdites cellules de myélome pendant un temps suffisant pour réaliser la liaison et l'hybridation entre lesdites cellules de myélome et lesdites cellules produisant l'anticorps désiré ou les précurseurs des cellules; et à isoler les hybrides qui sont produits dans l'étape précédente et qui produisent l'anticorps spécifique désiré; caractérisé en ce que lesdites cellules de myélome ont l'antigène spécifique audit anticorps désiré sur leur surface ou comme partie de leur surface.

2. Un procédé selon la revendication 1, caractérisé en ce que ladite étape d'isolement comprend l'isolement de tous les hybrides entre lesdites cellules de myélome et lesdites cellules normales puis l'isolement à partir de là des hybrides produisant ledit anticorps désiré.

3. Un procédé selon la revendication 1 ou la revendication 2, caractérisé en ce que ladite population de cellules normales constituée de cellules produisant l'anticorps désiré ou des précurseurs de ces cellules est obtenue par un des procédés suivants:

(a) immunisation d'un organisme avec l'antigène spécifique à l'anticorps désiré et isolement des cellules à partir dudit organisme;

(b) isolement des cellules produites par un organisme en réponse à un état maladif qui manifeste l'antigène spécifique audit anticorps désiré;

(c) stimulation des cellules normales d'un organisme in vitro avec l'antigène spécifique à l'anticorps désiré;

(d) traitement des cellules normales d'un organisme in vitro avec une substance chimique capable de stimuler lesdites cellules normales pour produire l'anticorps désiré.

4. Un procédé selon l'une quelconque des revendications précédentes caractérisé en ce que lesdites cellules de myélome sont obtenues par fixation dudit antigène à la surface d'une cellule de myélome normale par un des procédé suivants.

(a) Fixation dudit antigène à la surface desdites cellules normales de myélome par fixation chimique covalente;

(b) mise en contact des dites cellules normales de myélome avec ledit antigène pour réaliser l'adsorption du dit antigène à la surface de celles-ci;

(c) fixation de l'antigène à un moyen de pontage capable de le fixer à des membranes de la surface des cellules;

(d) incubation desdites cellules normales de myélome avec des complexes de l'antigène et de l'anticorps désiré pour réaliser une liaison entre le myélome et le complexe; et

(e) incorporation de l'antigène sur ou au sein d'un liposome qui fusionne avec la membrane de ladite cellule normale de myélome pour déposer les produits du liposome sur la surface de la cellule de myélome.

5. Un procédé selon la revendication 1, caractérisé en ce que lesdites cellules de myélome sont fournies en induisant les cellules normales de myélome à avoir à leur surface l'antigène requis.

6. Un procédé selon la revendication 5, caractérisé en ce que ladite étape d'induction comprend l'hybridation de la cellule normale de myélome avec une cellule cancéreuse ayant l'antigène spécifique à l'anticorps désiré.

7. Un procédé selon la revendication 1, caractérisé en ce que l'antigène ou l'anticorps spécifique sont aussi mélanges avec ladite population de cellules normales et de cellules de myélome dans ladite étape de mélange inhibant ainsi toute liaison spécifique entre les cellules de myélome et les cellules produisant l'anticorps désiré sauf entre les cellules de myélome et les cellules qui produisent l'anticorps désiré et qui ont l'affinité la plus élevée pour l'antigène présent sur lesdites cellules de myélome.